# EUROPÄISCHE PATENTSCHRIFT

(11) **EP 1 937 615 B1**
(45) Veröffentlichungstag und Bekanntmachung des Hinweises auf die Patenterteilung: **09.08.2017**
(21) Anmeldenummer: 06792129.6
(22) Anmeldetag: 19.09.2006
(51) Int. Cl.: C07C 1/20, C07C 11/06, B01D 3/14

(54) **VERFAHREN ZUR HERSTELLUNG VON C2-C4-OLEFINEN AUS METHANOL UND/ODER DIMETHYLETHER**
METHOD FOR PRODUCING C2-C4-OLEFINS FROM METHANOL AND/OR DIMETHYL-ETHER
PROCEDE POUR PRODUIRE DES OLEFINES C2-C4 A PARTIR DE METHANOL ET/OU DE DIMETHYLETHER

(30) Priorität: 13.10.2005 DE 102005048931
(43) Veröffentlichungstag der Anmeldung: 02.07.2008
(73) Patentinhaber: Air Liquide Global E&C Solutions Germany GmbH, 60439 Frankfurt am Main (DE)
(72) Erfinder: ROTHAEMEL, Martin, 58452 Witten (DE); BUCHHOLD, Henning, 63452 Hanau (DE); KÖMPEL, Harald, 63263 Neu-Isenburg (DE); GLASMACHER, Andreas, 53123 Bonn (DE); OCHS, Andreas, 61381 Friedrichsdorf (DE)
(74) Vertreter: Keil & Schaafhausen Patent- und Rechtsanwälte PartGmbB
(86) Internationale Anmeldenummer: PCT/EP2006/009076
(87) Internationale Veröffentlichungsnummer: WO 2007/042124

(56) Entgegenhaltungen:
- EP-A1- 0 882 692
- DE-A1- 10 027 159
- DE-A1- 10 233 975

## Beschreibung

Die vorliegende Erfindung betrifft ein Verfahren zur Herstellung von C₂-C₄-Olefinen, insbesondere von Propylen, aus einem Wasserdampf und Oxygenate, wie Methanol und/oder Dimethylether, enthaltenden Eduktgemisch, bei dem das Eduktgemisch in wenigstens einem Reaktor an einem Katalysator zu einem niedermolekulare Olefine und Benzinkohlenwasserstoffe umfassenden Reaktionsgemisch umgesetzt wird, welches in einer ersten Trenneinrichtung in ein C₅₋-olefinreiches Gemisch, ein an C₅₊-Benzinkohlenwasserstoffen reiches Gemisch und eine wässrige Phase aufgetrennt wird.

Zur Herstellung von niedermolekularen C₂-C₄-Olefinen, insbesondere Propylen, aus Methanol und/oder Dimethylether, sind dem Fachmann eine Vielzahl an Verfahren bekannt, welche üblicherweise auf der Umsetzung eines Wasserdampf sowie Methanol- und/oder Dimethyletherdampf enthaltenden Eduktgemischs an einem formselektiven Zeolith-Katalysator basieren.

So beschreibt die DE 100 27 159 A1 ein Verfahren zur Herstellung von Propylen aus Methanol, bei dem zunächst aus Methanoldampf an einem ersten Katalysator eine Dimethylether enthaltendes Dampfgemisch erzeugt wird, bevor dieses mit Wasserdampf vermischt und in wenigstens zwei in Reihe geschalteten Schachtreaktoren mit Katalysatorschüttungen aus formselektivem Zeolith zu einem propylenhaltigen Produktgemisch umgesetzt wird. Anschließend wird das Produktgemisch in einer mehrere Destillationskolonnen umfassenden Trenneinrichtung aufgearbeitet, wobei eine propylenreiche Fraktion mit einem Propylengehalt von wenigstens 95 Vol.-%, eine niedermolekulare Kohlenwasserstoffe enthaltende Fraktion, welche in die Katalysatorschüttungen zurückgeführt wird, und eine benzinkohlenwasserstoffreiche Fraktion, welche aus dem Verfahren entfernt wird, erhalten wird. Nachteilig an diesem Verfahren ist jedoch die, bezogen auf den Gesamtkohlenstoffgehalt in dem Eduktgemisch, geringe Ausbeute an Propylen, die u.a. dadurch verursacht wird, dass die benzinkohlenwasserstoffreiche Fraktion ungenutzt aus dem Verfahren entfernt wird.

Aus der EP 0 882 692 B1 ist ein Verfahren zur Herstellung von C₂-C₃-Olefinen bekannt, bei dem ein Gemisch aus Wasserdampf und Methanol- und/oder Dimethyletherdampf in einem einen Zeolith-Katalysator enthaltenden Röhrenreaktor bei einer Temperatur zwischen 280 und 570°C und einem Druck zwischen 0,1 und 0,9 bar zu einem olefinreichen Produktgemisch umgesetzt wird, welches anschließend in einer Trenneinrichtung in eine C₂-C₄-Olefin-Fraktion mit einem Propylengehalt von wenigstens 40 Gew.-%, eine wasserhaltige Fraktion, eine gasförmige Fraktion und eine C₅₊-Benzinkohlenwasserstoffe enthaltende Fraktion aufgetrennt wird. Während die drei erstgenannten Fraktionen aus dem Verfahren abgezogen werden, wird der die C₅₊-Benzinkohlenwasserstoffe enthaltende Produktstrom mit Wasser vermischt, in einem Erhitzer auf eine Temperatur von 380 bis 700°C erhitzt und in einem zweiten, einen Zeolith-Katalysator enthaltenden Reaktor zu C₂-C₄-Olefinen umgesetzt, bevor die Reaktionsprodukte zu der Trenneinrichtung zurückgeführt werden. Die mit diesem Verfahren erhaltenen Ausbeuten an C₂-C₃-Olefinen sind, obgleich höher als bei dem aus der DE 100 27 159 A1 bekannten Verfahren, ebenfalls verbesserungsbedürftig. Zudem zeichnet sich dieses Verfahren nicht zuletzt wegen der isothermen Verfahrensführung sowie dem notwendigen Vakuumbetrieb in dem Röhrenreaktor durch hohe Kosten aus.

Bei den bekannten Verfahren fällt am Reaktoraustritt nach der Kondensation außer dem propylenreichen Gasgemisch auch ein flüssiges Kohlenwasserstoffprodukt an, welches aus Olefinen, Paraffinen, Naphthenen und Aromaten besteht. Grundsätzlich wäre es denkbar, zur Steigerung der Ausbeute dieses Flüssigprodukt in den Reaktor zurückzuführen, um die enthaltenen Olefine und Naphthene selektiv in Propylen umzusetzen. Die hierbei jedoch zwangsläufig ebenfalls zurückgeführten Aromaten, die den überwiegenden Anteil des Flüssigproduktes ausmachen, reagieren jedoch mit dem dem Reaktor als Feed zugeführten Methanol, bspw. durch Alkylierung von Benzol zu Toluol, von Toluol zu Xylolen usw. Da hierdurch weniger Methanol für die selektive Umsetzung von Propylen zur Verfügung steht, wird die erreichbare Ausbeute geschmälert.

Im Stand der Technik sind verschiedene Verfahren bekannt, um Aromaten (Benzol, Toluol und Xylole) aus Kohlenwasserstoff-Strömen abzutrennen. Während lange Zeit die Flüssig-Flüssig-Extraktion das bevorzugte Verfahren zur Rückgewinnung von Aromaten war, wurde jüngst eine Extraktiv-Destillation vorgeschlagen, mittels der auch Mischungen aufgetrennt werden können, deren Komponenten nur gering unterschiedliche Siedepunkte aufweisen. Hierbei wird durch spezielle Lösungsmittel der Volatilitätsunterschied zwischen den zu trennenden Komponenten erhöht. Das Extraktionsmittel und die weniger flüchtige Komponente fließen zum Boden der Destillationskolonne, wo die extrahierte Komponente durch eine weitere Destillation gewonnen wird. Die nicht extrahierte Komponente wird am oberen Ende der Extraktionsdestillationskolonne abdestilliert. Zur Schwefelreduzierung in Kraftstoffen wurde dieses GT DeSulf-Verfahren bspw. auf dem ERTC 7th Annual Meeting, Paris, Frankreich, 18. bis 20. November 2002 von Lucia Cretoiu, Josepf C. Gentry, Sam Kumar und Randi Wright-Wytcherley "Sulfur Reduction With No Octane Loss - GT DeSulf" oder auf dem 2003 AlChE Spring Meeting, New Orleans, USA, 1. bis 2. April 2003 von Joseph Gentry, Sam Kumar und Randi Wright-Wytcherley "Extractive Distillation Applied" vorgestellt.

Zur Reduzierung des Benzolgehaltes in Kraftstoffen wurde zudem die Abtrennung mittels hochleistungsfähiger Pervaporationsmembranen vorgeschlagen (vgl. "Benzolgehalt in Kraftstoffen reduzieren", CIT plus 10/2004, Seite 45) oder "Pervaporation shows promise for separating benzene from aliphatics", Chemical Engineering 9/2004).

Aufgabe der Erfindung ist es, bei einem gattungsgemäßen Verfahren die Ausbeute an C₂-C₄-Olefinen, insbesondere von Propylen, zu erhöhen.

Diese Aufgabe wird mit der Erfindung durch die Merkmale des Anspruchs 1 gelöst. Im Wesentlichen wird das an C₅₊-Benzinkohlenwasserstoffen reiche Gemisch einer zweiten Trenneinrichtung zugeführt wird, in welcher die in dem Gemisch enthaltenen Aromaten weitgehend abgetrennt und als Aromatenstrom abgeführt werden, und dass der weitgehend aromatenfreie Reststrom als Recyclingstrom wenigstens teilweise zu dem wenigstens einen ersten Reaktor zurückgeführt wird.

Mit der Erfindung lässt sich das bekannte Verfahren gleich in zweifacher Weise verbessern. Zum einen kann das von Aromaten befreite Flüssigprodukt nun vollständig in den Reaktor zurückgeführt werden, wo sich der Olefin- und Naphthen-Anteil größtenteils zu Propylen umsetzen und damit die Ausbeute der Gasamtanlage erhöhen lässt. Zum anderen wird zusätzlich ein wertvolles Gemisch aus Reinaromaten erzeugt, das im Vergleich zum ursprünglichen Benzinprodukt einen deutlich höheren Wert aufweist.

Nachdem sich eine 100 %-ige Abtrennung der Aromaten nicht realisieren lässt, weist erfindungsgemäß der Anteil an Aromaten in dem zu dem ersten Reaktor zurückgeführten Recyclingstrom einen Anteilunter 5 Gew.-% und insbesondere von weniger als 1 Gew.-% auf.

Um die Aufkonzentration insbesondere der reaktionsträgen Paraffine im Kreislauf zu kontrollieren, wird in Weiterbildung der Erfindung ein Teil des in der zweiten Trenneinrichtung erhaltenen, weitgehend aromatenfreien Recyclingstroms aus dem Prozess entfernt.

Um den wirtschaftlichen Wert des Aromatengemisches zu erhöhen, wird der in der zweiten Trenneinrichtung abgetrennte Aromatenstrom erfindungsgemäß in einer weiteren Trenneinrichtung in Benzol, Toluol und ein Xylol-Isomerengemisch aufgetrennt.

Vorzugsweise ist der Reaktor als Schachtreaktor, Röhrenreaktor, stationärer Wirbelschichtreaktor oder zirkulierender Wirbelschichtreaktor ausgebildet. Im zweitgenannten Fall sind in dem Reaktor vorzugsweise mehrere axial angeordnete Röhren vorgesehen, welche bspw. eine Länge zwischen 1 und 5 m sowie einen Innendurchmesser von 20 bis 50 mm aufweisen.

Um eine möglichst hohe Umsetzung des Eduktgemischs zu erreichen, wird dieses gemäß einer besonderen Ausführungsform der vorliegenden Erfindung durch zwei oder mehr hintereinander geschaltete Reaktoren geführt. Für diese Ausführungsform haben sich insbesondere mehr als zwei, bevorzugt vier, in Serie geschaltete Schachtreaktoren jeweils mit einem formselektiven Zeolith-Katalysator als besonders geeignet erwiesen, wobei in dem ersten Schachtreaktor ein Teil des Eduktgemischs aus dem Vorreaktor und in jeden weiteren Schachtreaktor das Produktgemisch des diesem jeweils vorgeschalteten Schachtreaktors zusammen mit einem Teilstrom des Eduktgemischs aus dem Vorreaktor geleitet wird. Ebenso gute Umsetzungsgrade werden erhalten, wenn alternativ zu der vorgenannten Ausführungsform das Eduktgemisch durch nur einen Reaktor geführt wird, in dem wenigstens zwei hintereinander angeordneten Katalysatorstufen vorgesehen sind. In diesem Fall sind die einzelnen Katalysatorstufen vorzugsweise untereinander angeordnet und werden von dem Eduktgemisch von oben nach unten durchströmt. Auch hier wird das Eduktgemisch aus dem Vorreaktor auf die einzelnen Katalysatorstufen verteilt.

Grundsätzlich können in dem wenigstens einen Reaktor alle dem Fachmann zur Umsetzung von Methanol und/oder Dimethylether zu C₂-C₄-Olefinen geeignete Zeolith-Katalysatoren eingesetzt werden, wobei sich insbesondere Alumosilikat-Zeolith vom Pentasiltyp und besonders bevorzugt ZSM-5 als geeignet erwiesen haben. Zur Optimierung der Ausbeute wird dem Reaktor bzw. der ersten Reaktorstufe, vorzugsweise mindestens ein inerter Strom, besonders bevorzugt Wasserdampf, und mindestens ein Kohlenwasserstoffe enthaltender Strom zugeführt.

Um die Betriebskosten des Verfahrens abzusenken, wird in Weiterbildung des Erfindungsgedankens vorgeschlagen, die Umsetzung in dem wenigstens einen Reaktor adiabatisch durchzuführen. Alternativ kann eine isotherme Verfahrensführung vorgesehen sein, die jedoch im Vergleich zu der adiabatischen Verfahrensführung zu höheren Verfahrenskosten führt.

Gute Ausbeuten in dem wenigstens einen Reaktor werden insbesondere erhalten, wenn diesem ein Eduktgemisch mit einem Gewichtsverhältnis Wasser zu Methanoläquivalent von 0,25 : 1 bis 10 : 1 zugeführt wird. Sofern der Reaktor mehrere Katalysatorstufen umfasst, gilt dieses Verhältnis für den Eintritt in jede Katalysatorstufe. Ein "Methanoläquivalent" entspricht nach der Gleichung 2 CH₃OH → CH₃-O-CH₃ + H₂O einem halben Mol Dimethylether (DME). Zudem wird das Eduktgemisch in dem Reaktor bevorzugt bei einer Temperatur von 300 bis 600°C und/oder bei einem Druck von 0,5 bis 5 bara umgesetzt.

Zur Auftrennung des aus dem Reaktor abgezogenen Reaktionsgemischs kann jede dem Fachmann bekannte Trenneinrichtung eingesetzt werden, die geeignet ist, ein C₂-C₄-olefinreiches Gemisch von einem an C₅₊-Benzinkohlenwasserstoffen reichen Gemisch abzutrennen, bspw. destillativ, adsorptiv, thermisch oder mittels Membranen arbeitende Trenneinrichtungen.

Besonders gute Ergebnisse werden erhalten, wenn die erste Trenneinrichtung als Kühlvorrichtung ausgebildet ist und das aus dem ersten Reaktor abgezogene Reaktionsgemisch in dieser auf eine Temperatur von 10 bis 80°C abgekühlt wird.

Gemäß einer weiteren bevorzugten Ausführungsform der vorliegenden Erfindung wird das aus der ersten Trenneinrichtung abgezogene C₂-C₄-olefinreiche Gemisch einer dritten Trenneinrichtung zugeführt, in der das vorgenannte Gemisch in einen C₄-C₅-Olefin- und in einen C₃₋-Olefinstrom aufgetrennt wird. Dies ermöglicht die Rückführung des C₄-C₅-Olefinstroms in den Reaktor, womit die Gesamtausbeute des Verfahrens weiter gesteigert werden kann. Aus dem C₃₋-Olefinstrom kann Propylen bspw. destillativ in einfacher Weise und hoher Reinheit gewonnen werden. Vorzugsweise werden auch die nach dem Abtrennen von Propylen aus dem C₃₋-Olefinstrom verbleibenden Olefine in den Reaktor zurückgeführt. Offenbarungsgemäß umfasst die Anlage wenigstens einen katalytischen Reaktor zur Umsetzung des Eduktgemischs in ein niedermolekulare Olefine und Benzinkohlenwasserstoffe umfassendes Reaktionsgemisch sowie eine erste Trenneinrichtung zur Auftrennung des in dem Reaktor erhaltenen Reaktionsgemischs in ein C₅₋-olefinreiches Gemisch, ein an C₅₊-Benzinkohlenwasserstoffen reiches Gemisch und eine wässrige Phase, sowie eine zweite Trenneinrichtung, die daran angepasst ist, das an C₅₊-Benzinkohlenwasserstoffen reiche Gemisch in einen im Wesentlichen aromatenfreien Recyclingstrom und einen die abgetrennten Aromaten enthaltenden Aromatenstrom aufzutrennen, wobei eine Rückführleitung von der zweiten Trenneinrichtung zu dem Reaktor führt.

Gemäß einer bevorzugten Ausgestaltung der Offenbarung ist die zweite Trenneinrichtung eine Membrantrenneinrichtung.

Alternativ kann die zweite Trenneinrichtung auch eine Destillationskolonne aufweisen, in welcher vorzugsweise eine Extraktionsmittel zugesetzt wird.

In Weiterbildung der Offenbarung wird vorgeschlagen, der ersten Trenneinrichtung eine dritte Trenneinrichtung zur Auftrennung des aus der ersten Trenneinrichtung abgezogenen C₅₋-olefinreichen Gemischs in einen C₄-C₅-Olefin- und einen C₃₋-Olefinstrom nachgeschaltet ist, wobei die dritte Trenneinrichtung vorzugsweise wenigstens eine Destillationskolonne aufweist.

In Weiterbildung der Offenbarung ist eine von der dritten Trenneinrichtung zu dem Reaktor führende Rückführleitung vorgesehen, um das aus der dritten Trenneinrichtung abgezogene C₄-C₅-olefinreiche Gemisch in den Reaktor zurückzuführen.

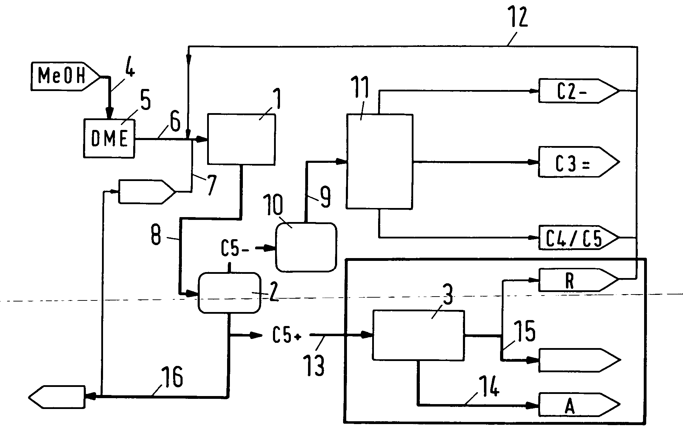

Weitere Merkmale, Vorteile und Anwendungsmöglichkeiten der Erfindung ergeben sich auch aus der nachfolgenden Beschreibung von Ausführungsbeispielen und der Zeichnung.

Die einzige Figur zeigt schematisch eine zur Durchführung des erfindungsgemäßen Verfahrens geeignete Anlage.

Die in der Figur dargestellte Anlage umfasst einen Reaktor 1 mit einem Katalysator auf Basis von formselektivem Zeolith, vorzugsweise ein Alumosilikat-Zeolith vom Pentasiltyp und besonders bevorzugt ZSM-5. Der Reaktor 1 ist vorzugsweise als Schachtreaktor, Röhrenreaktor, stationärer Wirbelschichtreaktor oder zirkulierender Wirbelschichtreaktor ausgestaltet. Des Weiteren umfasst die Anlage eine als Kühler ausgestaltete erste Trenneinrichtung 2 sowie eine zweite Trenneinrichtung 3.

Beim Betrieb der Anlage wird über eine Methanolzufuhrleitung 4 herangeführtes Methanol in einem nicht dargestellten Wärmeaustauscher auf eine Temperatur von vorzugsweise 200 bis 350°C erhitzt und dadurch verdampft, bevor der Methanoldampf in einem Vorreaktor 5 an einem geeigneten Dehydratisierungskatalysator, bspw. Aluminiumoxid, zumindest teilweise zu Dimethylether und Wasser umgesetzt wird. Dem über eine Leitung 6 aus dem Vorreaktor 5 abgezogenen Methanol/Dimethylethergemisch wird über eine Dampfleitung 7 Wasserdampf zugeführt und das so erhaltene Gemisch in den Reaktor 1 geleitet. Vorzugsweise beträgt die Eintrittstemperatur des Eduktgemischs in den Reaktor 1 zwischen 350 und 500°C, das Gewichtsverhältnis Wasser zu Methanoläquivalent in dem Eduktgemisch zwischen 0,25 : 1 und 10 : 1 sowie der Druck in dem Reaktor 1 zwischen 0,5 und 5,0 bara. Im Katalysatorbereich des Reaktors 1 liegen die Temperaturen bevorzugt zwischen 300 und 600°C.

Alternativ zu der in der Figur dargestellten einstufigen Ausgestaltung des Reaktors 1 kann dieser auch aus zwei oder mehr in Serie geschalteten Reaktionsstufen stehen, wobei es sich dabei sowohl um separat ausgeführte Reaktoren als auch um übereinander angeordnete Katalysatorschüttungen in einem Reaktor handeln kann. In diesem Fall wird das Produkt aus dem Vorreaktor 5 auf die einzelnen Stufen verteilt, während alle anderen Eintrittsströme komplett in die erste Reaktionsstufe eingeleitet werden. Ferner ist es möglich, anstelle eines Wasserdampf-/Methanol-/Dimethylethergemischs in den Reaktor 1 ausschließlich Methanol oder Dimethylether in Kombination mit Wasserdampf als Edukt einzusetzen.

Über eine Leitung 8 wird das in dem Reaktor 1 gebildete und vornehmlich aus C₂-C₄-Olefinen, C₅₊-Benzinkohlenwasserstoffen und Wasserdampf bestehende Reaktionsgemisch aus dem Reaktor 1 abgezogen und der ersten Trenneinrichtung 2 zugeführt, in welcher das Reaktionsgemisch auf eine Temperatur zwischen 10 und 80°C abgekühlt wird, so dass ein wasserreiches Kondensat, eine flüssige organische Phase, welche reich an C₅₊-Benzinkohlenwasserstoffen ist, sowie eine im Wesentlichen aus C₂-C₄-Olefinen bestehende gasförmige C₅₋-olefinreiche Fraktion erhalten wird.

Aus der ersten Trenneinrichtung 2 wird die C₅₋-olefinreiche Fraktion über eine Leitung 9 nach Durchlaufen eines Verdichters 10 einer dritten Trenneinrichtung 11 zugeführt, in welcher sie in einen C₄-C₅-Kohlenwasserstoff-, einen C₂₋-Kohlenwasserstoff- und einen Propylenstrom aufgetrennt wird. Es ist auch möglich, zunächst eine Abtrennung eines C₃₋-Olefinstroms vorzunehmen und aus diesem anschließend in bekannter weise das Propylen zu gewinnen. Der C₄-C₅-Kohlenwasserstoffstrom und der C₂₋- Kohlenwasserstoffstrom werden über eine gemeinsame oder separate Rückführleitung(en) 12 in die Leitung 6 und zusammen mit dem Methanol-/Dimethylethergemisch und dem Wasserdampf in den Reaktor 1 eingeführt.

Die in der ersten Trenneinrichtung 2 gewonnene, im Wesentlichen aus C₅₊-Benzinkohlenwasserstoffen bestehende organische Phase wird über eine Leitung 13 in die zweite Trenneinrichtung 3 eingeführt, in welcher die in dem Flüssigprodukt enthaltenen Aromaten praktisch vollständig abgetrennt werden. Die zweite Trenneinrichtung 3 ist hierzu als Membrantrenneinrichtung ausgestaltet oder trennt die Aromaten in einer Extraktivdestillation unter Verwendung spezieller Extraktionsmittel ab. Es ist auch möglich, die vorgenannten Verfahren in Kombination und ggf. auch in Verbindung mit einer Flüssig-Flüssig-Destillationsvorrichtung einzusetzen. Diese Verfahren sind dem Fachmann grundsätzlich bekannt. Es wird insoweit auf die in der Beschreibungseinleitung zitierten Dokumente verwiesen. Durch die zweite Trenneinrichtung 3 werden die Aromaten soweit abgetrennt, dass in dem Reststrom weniger als 5 Gew.-%, vorzugsweise weniger als 1 Gew.-% Aromaten verbleiben. Der abgetrennte Aromatenstrom A wird über eine Leitung 14 abgeführt und ggf. in einer nicht dargestellten weiteren Trenneinrichtung nach dem Fachmann bekannten Methoden in Benzol, Toluol und ein Xylol-Isomerengemisch weiter aufgetrennt.

Der weitgehend aromatenfreie Reststrom wird als Recyclingstrom R bspw. über die Rückführleitung 12 zu dem Reaktor 1 zurückgeführt.

Um eine Aufkonzentration insbesondere der reaktionsträgen Paraffine im Kreislauf zu vermeiden, kann ein Teil des aromatenfreien Reststroms von dem Recyclingstrom R abgetrennt und über eine Leitung 15 aus dem Prozess entfernt werden.

Die in der ersten Trenneinrichtung 2 abgetrennte wässrige Phase kann über eine Leitung 16 abgeführt und nach Umwandlung in Dampf ggf. teilweise über die Leitung 7 wieder in den Prozess zurückgeführt werden.

### Beispiel

Ein Methanol-Einsatz von 208.3 t/h führt bei einer Jahreslaufzeit von 8000 h zu einem Zufuhrstrom von 1,66 Mio t/a. Da bei der Umsetzung von Methanol zwangsläufig Wasser frei wird, beziehen sich die prozentualen Produktausbeuten auf den so genannten "CH₂"-Anteil (aus der Pseudoreaktion CH₃OH → "CH₂" + H₂O). Der Zufuhrstrom beträgt für den "CH₂"-Anteil 91.2 t/h (730 kt/a).

Bei einem Vergleich der Produktverteilung des herkömmlichen Verfahrens (MTP), wie es bspw. in der EP 0 882 692 B1 beschrieben ist, mit dem erfindungsgemäßen Verfahren (MTP+) ergaben sich für den oben genannten Methanol-Einsatz folgende Produktströme:

| Produkte [kt/a] | Herkömmliches Verfahren (MTP) | Erfindungsgemäßes Verfahren (MTP+) |
|---|---|---|
| Propylen | 450 (61.7 %) | 477 |
| Benzin | 214 (26.8 %) | 81 |
| Aromaten | 0 | 102 |
| Andere (C2-, LPG) | 65 (8.9 %) | 69 |

Es ergibt sich mit dem erfindungsgemäßen Verfahren eine um 6 % erhöhte Propylenproduktion. Außerdem werden Aromaten als separate Produkte erzeugt, die als Einzelkomponenten einen höheren Verkaufspreis erzielen als das herkömmlicherweise erhaltene Benzinprodukt.

Gleichzeitig ändert sich auch die Zusammensetzung des Benzins

| Benzinzusammensetzung [Gew-%] | MTP | MTP+ |
|---|---|---|
| Olefine | 19 | 18 |
| Naphtene | 13 | 16 |
| Aromaten | 46 | 4 |
| Paraffine | 12 | 62 |

Der weitgehend aromatenfreie Recyclingstrom kann sich im Reaktor umsetzen. Hierbei sind aber nur die Olefine (Umsatz ca. 86 %) und die Naphtene (Umsatz ca. 58 %) reaktiv, während Paraffine inert sind. Entsprechend ist zu erkennen, dass sich die Paraffine gegenüber dem Benzinstrom stark angereichert haben. Der Aromatenanteil beträgt nur noch 4 %. Die Paraffine werden daher abgeführt, wobei insbesondere ein Purge-Anteil von 30 % festgelegt werden kann.

Der wesentlich verringerte Aromatengehalt erhöht den Wert des erhaltenen Benzins, insbesondere nachdem in der Europäischen Union ab 2006 für Fahrbenzin ein maximaler Aromatengehalt von 35 Gew.-% vorgeschrieben ist.

### Bezugszeichenliste:

- 1: Reaktor
- 2: erste Trenneinrichtung
- 3: zweite Trenneinrichtung
- 4: Methanolzufuhrleitung
- 5: Vorreaktor
- 6: Leitung
- 7: Dampfleitung
- 8: Leitung
- 9: Leitung
- 10: Verdichter
- 11: dritte Trenneinrichtung
- 12: Rückführleitung
- 13: Leitung
- 14: Leitung
- 15: Leitung
- 16: Leitung

- A: Aromatenstrom
- R: Recyclingstrom

## Patentansprüche

1. Verfahren zur Herstellung von C₂-C₄-Olefinen, insbesondere von Propylen, aus einem Wasserdampf und Oxygenate, wie Methanol und/oder Dimethylether, enthaltenden Eduktgemisch, bei dem das Eduktgemisch in wenigstens einem Reaktor (1) an einem Katalysator zu einem niedermolekulare Olefine und Benzinkohlenwasserstoffe umfassenden Reaktionsgemisch umgesetzt wird, welches in einer ersten Trenneinrichtung (2) in ein C₅₋-olefinreiches Gemisch, ein an C₅₊-Benzinkohlenwasserstoffen reiches Gemisch und eine wässrige Phase aufgetrennt wird,
**dadurch gekennzeichnet, dass** das an C₅₊-Benzinkohlenwasserstoffen reiche Gemisch einer zweiten Trenneinrichtung (3) zugeführt wird, in welcher die in dem Gemisch enthaltenen Aromaten weitgehend abgetrennt und als Aromatenstrom (A) abgeführt werden, und
dass der eine Aromatenanteil unter 5 Gew.-% aufweisende Reststrom als Recyclingstrom (R) wenigstens teilweise zu dem wenigstens einen Reaktor (1) zurückgeführt wird.

2. Verfahren nach Anspruch 1, **dadurch gekennzeichnet, dass** der Anteil an Aromaten in dem zu dem ersten Reaktor (1) zurückgeführten Recyclingstrom (R) unter 1 Gew.-% liegt.

3. Verfahren nach einem der vorhergehenden Ansprüche, **dadurch gekennzeichnet, dass** ein Teil des in der zweiten Trenneinrichtung (3) erhaltenen weitgehend aromatenfreien Recyclingstroms (R) aus dem Prozess entfernt wird.

4. Verfahren nach einem der vorhergehenden Ansprüche, **dadurch gekennzeichnet, dass** der in der zweiten Trenneinrichtung (3) abgetrennte Aromatenstrom (A) in einer weiteren Trenneinrichtung in Benzol, Toluol und ein Xylol-Isomerengemisch aufgetrennt wird.

5. Verfahren nach einem der vorhergehenden Ansprüche, **dadurch gekennzeichnet, dass** der wenigstens eine Reaktor (1) als Schachtreaktor, Röhrenreaktor, stationärer Wirbelschichtreaktor oder zirkulierender Wirbelschichtreaktor ausgebildet ist.

6. Verfahren nach einem der vorhergehenden Ansprüche, **dadurch gekennzeichnet, dass** zwei oder mehr hintereinander geschaltete Reaktoren (1) eingesetzt werden oder ein Reaktor (1) umfassend wenigstens zwei hintereinander angeordnete Katalysatorstufen eingesetzt wird.

7. Verfahren nach einem der vorhergehenden Ansprüche, **dadurch gekennzeichnen,** dass der Katalysator in dem Reaktor (1) ein körniger formselektiver Zeolith-Katalysator, insbesondere ein Alumosilikat-Zeolith vom Pentasiltyp, bevorzugt ZSM-5, ist.

8. Verfahren nach einem der vorhergehenden Ansprüche, **dadurch gekennzeichnet, dass** die Umsetzung in dem wenigstens einen Reaktor (1) adiabatisch durchgeführt wird.

9. Verfahren nach einem der vorhergehenden Ansprüche, **dadurch gekennzeichnet, dass** die erste Trenneinrichtung (2) eine Kühlvorrichtung ist, in der das Reaktionsgemisch auf eine Temperatur von 10 bis 80°C abgekühlt wird.

10. Verfahren nach einem der vorhergehenden Ansprüche, **dadurch gekennzeichnet, dass** das in der ersten Trenneinrichtung (2) erhaltene C₅₋-olefinreiche Gemisch einer dritten Trenneinrichtung (11) zugeführt wird, in der das Gemisch in einen C₄-C₅-Kohlenwasserstoff-, einen C₂₋-Kohlenwasserstoff- und einen C₃-Kohlenwasserstoffstrom aufgetrennt wird.

11. Verfahren nach Anspruch 10, **dadurch gekennzeichnet, dass** der C₂₋-Kohlenwasserstoffstrom in den Reaktor (1) zurückgeführt wird.

12. Verfahren nach einem der Ansprüche 1 bis 9, **dadurch gekennzeichnet, dass** das in der ersten Trenneinrichtung (2) erhaltene C₅₋-olefinreiche Gemisch einer dritten Trenneinrichtung (11) zugeführt wird, in der das Gemisch in einen C₄-C₅-Kohlenwasserstoff- und einen C₃₋-Kohlenwasserstoffstrom aufgetrennt wird.

13. Verfahren nach einem der Ansprüche 10 bis 12, **dadurch gekennzeichnet, dass** der C₄-C₅- Kohlenwasserstoffstrom in den Reaktor (1) zurückgeführt wird.

14. Verfahren nach Anspruch 12, **dadurch gekennzeichnet, dass** aus dem C₃₋- Kohlenwasserstoffstrom Propylen abgetrennt wird und ggf. die verbleibenden Olefine in den Reaktor (1) zurückgeführt werden.

## Claims

1. A process for producing C₂-C₄ olefins, in particular propylene, from an educt mixture containing steam and oxygenates, such as methanol and/or dimethyl ether, in which the educt mixture is reacted in at least one reactor (1) on a catalyst to obtain a reaction mixture comprising low-molecular olefins and gasoline hydrocarbons, which in a first separating device (2) is separated into a mixture rich in C₅₋olefins, a mixture rich in C₅₊ gasoline hydrocarbons, and an aqueous phase,
**characterized in that** the mixture rich in C₅₊ gasoline hydrocarbons is supplied to a second separating device (3), in which the aromatics contained in the mixture are largely separated and are discharged as aromatics stream (A), and
that the residual stream featuring an amount of aromatics less than 5 wt-%, is at least partly recirculated to the at least one reactor (1) as recycling stream (R).

2. The process as claimed in claim 1, **characterized in that** the amount of aromatics in the recycling stream (R) recirculated to the first reactor (1) is less than 1 wt-%.

3. The process as claimed in any of the preceding claims, **characterized in**
**that** part of the recycling stream (R) largely free from aromatics, which was obtained in the second separating device (3), is removed from the process.

4. The process as claimed in any of the preceding claims, **characterized in**
**that** the aromatics stream (A) separated in the second separating device (3) is separated into benzene, toluene and a xylene-isomer mixture in a further separating device.

5. The process as claimed in any of the preceding claims, **characterized in**
**that** the at least one reactor (1) constitutes a shaft reactor, tubular reactor, stationary fluidized-bed reactor or circulating fluidized-bed reactor.

6. The process as claimed in any of the preceding claims, **characterized in**
**that** two or more series-connected reactors (1) are used or one reactor (1) comprising at least two series-connected catalyst stages is used.

7. The process as claimed in any of the preceding claims, **characterized in**
**that** the catalyst in the reactor (1) is a granular, form-selective zeolite catalyst, in particular an alumosilicate zeolite of the pentasil type, preferably ZSM-5.

8. The process as claimed in any of the preceding claims, **characterized in**
**that** the conversion in the at least one reactor (1) is performed adiabatically.

9. The process as claimed in any of the preceding claims, **characterized in**
**that** the first separating device (2) is a cooling device, in which the reaction mixture is cooled to a temperature of 10 to 80°C.

10. The process as claimed in any of the preceding claims, **characterized in**
**that** the mixture rich in C₅₋ olefins, which was obtained in the first separating device (2), is supplied to a third separating device (11), in which the mixture is separated into a C₄-C₅ hydrocarbon stream, a C₂₋ hydrocarbon stream and a C₃₋ hydrocarbon stream.

11. The process as claimed in claim 10, **characterized in that** the C₂₋ hydrocarbon stream is recirculated to the reactor (1).

12. The process as claimed in any of claims 1 to 9, **characterized in that** the mixture rich in C₅₋ olefins, which was obtained in the first separating device (2), is supplied to a third separating device (11), in which the mixture is separated into a C₄-C₅ hydrocarbon stream and a C₃₋ hydrocarbon stream.

13. The process as claimed in any of claims 10 to 12, **characterized in that** the C₄-C₅ hydrocarbon stream is recirculated to the reactor (1).

14. The process as claimed in claim 12, **characterized in that** propylene is separated from the C₃₋ hydrocarbon stream and the remaining olefins are possibly recirculated to the reactor (1).

## Revendications

1. Procédé destiné à la préparation d'oléfines en C₂ à C₄, notamment de propylène, à partir d'un mélange d'éduits contenant de la vapeur d'eau et des produits d'oxygénation, comme du méthanol et/ou de l'éther diméthylique, lors duquel on transforme le mélange d'éduits dans au moins un réacteur (1) sur un catalyseur en un mélange réactionnel comprenant des oléfines de faible poids moléculaire et des hydrocarbures benzéniques, que l'on sépare dans un premier dispositif de séparation (2) en un mélange riche en oléfines en C₅, en un mélange riche en hydrocarbures benzéniques en C₅₊ et en une phase aqueuse,
**caractérisé en ce qu'**on amène le mélange riche en hydrocarbures benzéniques en C₅₊ vers un deuxième dispositif de séparation (3), dans lequel on sépare largement les aromates contenus dans le mélange et on les évacue sous la forme d'un flux d'aromates (A) et **en ce qu'**on ramène au moins partiellement le flux résiduel comportant une part d'aromates inférieure à 5 % en poids en tant que flux de recyclage (R) vers l'au moins un réacteur (1).

2. Procédé selon la revendication 1, **caractérisé en ce que** la part d'aromates dans le flux de recyclage (R) ramené vers le premier réacteur (1) est inférieure à 1 % en poids.

3. Procédé selon l'une quelconque des revendications précédentes, **caractérisé en ce qu'**on retire du processus une part du flux de recyclage (R) largement exempt d'aromates contenu dans le deuxième dispositif de séparation (3).

4. Procédé selon l'une quelconque des revendications précédentes, **caractérisé en ce que** dans un dispositif de séparation supplémentaire, on sépare le flux d'aromates (A) séparé dans le deuxième dispositif de séparation (3) en benzène, en toluène et un mélange d'isomères de xylènes.

5. Procédé selon l'une quelconque des revendications précédentes, **caractérisé en ce que** l'au moins un réacteur (1) est conçu en tant que réacteur à puits, réacteur à tubes, réacteur à lit fluidisé stationnaire ou réacteur à lit fluidisé circulant.

6. Procédé selon l'une quelconque des revendications précédentes, **caractérisé en ce qu'**on utilise deux réacteurs (1) montés l'un derrière l'autre ou plus, ou un réacteur (1) comprenant au moins deux étages catalyseurs, placés l'un derrière l'autre.

7. Procédé selon l'une quelconque des revendications précédentes, **caractérisé en ce que** le catalyseur dans le réacteur (1) est un catalyseur granuleux zéolithique à sélectivité de forme, notamment une zéolithe d'aluminosilicate de type pentasile, de préférence de la ZSM-5.

8. Procédé selon l'une quelconque des revendications précédentes, **caractérisé en ce que** la transformation dans l'au moins un réacteur (1) est réalisée de manière adiabatique.

9. Procédé selon l'une quelconque des revendications précédentes, **caractérisé en ce que** le premier dispositif de séparation (2) est un dispositif de refroidissement, dans lequel on refroidit le mélange réactionnel à une température de 10 à 80° C.

10. Procédé selon l'une quelconque des revendications précédentes, **caractérisé en ce qu'**on amène le mélange riche en oléfines en C₅ obtenu dans le premier dispositif de séparation (2) vers un troisième dispositif de séparation (11), dans lequel on sépare le mélange en un flux d'hydrocarbure en C₄ à C₆, un flux d'hydrocarbure en C₂ et en un flux d'hydrocarbure en C₃.

11. Procédé selon la revendication 10, **caractérisé en ce qu'**on ramène le flux d'hydrocarbure en C₂ dans le réacteur (1).

12. Procédé selon l'une quelconque des revendications 1 à 9, **caractérisé en ce qu'**on amène le mélange riche en oléfines en C₅ obtenu dans le premier dispositif de séparation (2) dans un troisième dispositif de séparation (11), dans lequel on sépare le mélange en un flux d'hydrocarbure en C₄ à C₅ et en un flux d'hydrocarbure en C₃.

13. Procédé selon l'une quelconque des revendications 10 à 12, **caractérisé en ce qu'**on ramène le flux d'hydrocarbure en C₄ à C₅ dans le réacteur (1).

14. Procédé selon la revendication 12, **caractérisé en ce qu'**on sépare à partir du flux d'hydrocarbure en C₃ du polypropylène et on ramène le cas échéant les oléfines restantes dans le réacteur (1).
